# EUROPEAN PATENT APPLICATION

(11) **EP 2 705 788 A1**
(43) Date of publication of application: **12.03.2014**
(21) Application number: 12779476.6
(22) Date of filing: 25.04.2012
(51) Int. Cl.: A61B 5/0452, A61B 5/0436

(54) **METHOD FOR MEASURING THE QT, QRS AND ST-T-INTERVALS OF THE CARDIO CYCLE AND DEVICE FOR CARRYING OUT SAID METHOD**

(30) Priority: 04.05.2011 UA 2011005650
(71) Applicant: Sosnytskyy, Volodymyr Mykolayovych, Kiev 02002 (UA); Sosnytska, Tayisiya Volodymyrivna, Kiev 02002 (UA)
(72) Inventor: Sosnytskyy, Volodymyr Mykolayovych, Kiev 02002 (UA); Sosnytska, Tayisiya Volodymyrivna, Kiev 02002 (UA)
(74) Representative: Office Freylinger
(86) International application number: PCT/UA2012/000047
(87) International publication number: WO 2012/150913

(57) **Abstract**

The invention pertains to the field of medicine, specifically, cardiology, and may be used in functional diagnosis of cardiovascular diseases.

According to the method, cardiac electromagnetic signals are recorded using the cardiac electromagnetic signals recording module, signals preliminary processing is then performed by averaging and preparing for analysis using the signals preliminary processing module; then data are analyzed and recognized using the signals transformation and analysis module, and final processing is performed using the QT, QRS, ST-T-of cardiac cycle intervals' duration calculation module, which serve as markers of myocardium's electric instability. During cardiac electromagnetic signals recording magnetic sensors of the cardiac electromagnetic signals recording module are used, isolating the magnetic component of cardiac electromagnetic signals, and magnetic field changes are recorded in one plane above the patient's chest, during data analysis and recognition using the signals transformation and analysis module single-step magnetic field maps are plotted using recorded and pre-processed heart's magnetic signals, and a "inverse problem solution" is solved, plotting single-step maps of current density vectors distribution of sources emitting the recorded magnetic signals, while every map is represented with a time interval not exceeding 4 milliseconds, and prior to final processing, using the global current density calculation module, single-step global current density values during the time interval of the whole cardiac cycle or its separate areas are calculated for each current density vectors distribution map, at final processing, calculating the duration of QT, QRS, ST-T-of cardiac cycle intervals; the time interval beginning and end are considered the point of time when the curve of global current density value change reaches or crosses the zero values line.

Device for measuring QT, QRS and ST-T-of cardiac cycle intervals contains successively connected cardiac electromagnetic signals recording module, signals preliminary processing module, artifacts removal, averaging, computer memory accumulation, including low and high frequency program filters, signals transformation and analysis module, global current density calculation module and QT, QRS and ST-T-intervals duration calculation module, while the cardiac electromagnetic signals recording module additionally contains magnetic sensors for recording the magnetic component of cardiac electromagnetic signals, and the signals transformation and analysis module is developed with a possibility to plot magnetic field maps and current density vectors distribution maps for each moment of the cardiac cycle.

Precision and reliability of cardiac cycle QT, QRS and ST-T-intervals were increased independent on the law of their changes and pathological lesions.

## Description

The invention pertains to the field of medicine, specifically of cardiology, and may be used in functional diagnostics of cardiovascular diseases.

The problem of treating cardiovascular diseases has been and remains one of the main priorities of scientists and doctors globally. In approximately 70% of cases death of ischemic heart disease is sudden. In 25-30% of cases sudden lethal heart failure is the first and last manifestation of ischemic heart disease. Timely forecasting of myocardium's electric instability, a key factor in analyzing death's arrhythmogenic mechanisms, becomes of special importance. Rather popular conventional methods: Holter electrocardiogram (ECG) monitoring, loading tests, echocardiography (ejection fraction) are still ineffective in establishing the risk degree of a specific patient's sudden death. There is the electrophysiological study and programmed stimulation method, but due to its complexity and risk for patients it cannot be recommended for wide use.

During the last decade, opportunities of the ventricle late potential (VLP) recording method have been studied actively. Heart late potentials are low-amplitude high-frequency signals after the QRS complex, covering the ST segments, reflecting ventricles' decelerated fragmented activity. An anatomical basis for late potentials is myocardium's heterogeneity: viable tissue alternates with necrosis and fibrosis areas, causing electric signals' asynchronous fragmentation, slowing of depolarization spread. Myocardium areas with decelerated ventricle depolarization may serve as substrate for re-entry - the main mechanism of ventricle tachycardia development, while ventricle late potentials are markers of this arrhythmogenic substrate.

However, based on an analysis of published literature and our own data, the listed methods' results have low specificity and forecasting value for both positive and negative results.

Pathological changes in myocardium are accompanied with various disorders of heart's electric activity, but are all characterized by joint regularities abnormal change of action current density and forecasting adverse with respect to lethally dangerous rhythm disorders. This forecasting-significant determination should form a basis for the methods of discovering and evaluating expressiveness of myocardium's electric instability.

As mentioned in numerous scientific literature sources and recommendations, in clinical conditions the only available method for analyzing myocardium's electric instability, reflecting its vulnerability with respect to development of life-threatening arrhythmias is evaluation of cardiac cycle QT, QRS and ST-T intervals.

Modem approaches to evaluation of myocardium's electric instability using measurement of cardiac cycle QT, QRS and ST-T intervals are described in the following sources:
Titce U.M., Shenk K. Semiconductor circuit technique. M.: Mir,1982.c.187-189;
O.V.Melnik New integrated methods of elements of electrocardiosignal morphology parameters estimation // ESBME 2006 Proceedings, 55.126;
RU No.2261653 C1, A61B 5/0452, 2005, Electrocardiosignal ST-segment extraction method and device for its performance;
Melnik O.V., Mikheyev A.A. Assessment of reliability of ST-segment form spectral indicators given the nature of choosing a transformation time window // International scientific and technical conference "Problems of information transmission and processing in telecommunication networks and systems". Conference materials. Ryazan, 2003;
RU No.2219828 C2, A61B 5/02, 2003, O. A. Zuikova, A. A. Mikheyev. Method of extracting of cardiac cycle beginning and suitable device;
Savelieva I. et al. "Agreement and reproducibility of automatic versus manual measurement of QT dispersion", American Journal of Cardiology, Cahners Publishing Co., Newton, MA, vol. 81, 1998, pp. 471-477;
Cohen T.J. et al. "A simple electrocardiographic algorithm for detecting ventricular tachycardia", PACE--Pacing and Clinical Electrophysiology, Futura Publishing Company, Inc., vol. 20, No. 10, Part 1, Oct. 1, 1997, pp. 2412-2418,
US Patent 5,560,370, A61B 005/468, 1996, Verrier et al. Method and apparatus for prediction of cardiac electrical instability by simultaneous assessment of T-wave alternans and QT interval dispersion - October;
US Patent 6,577,894 B2, A61B 5/05, 2003, Timothy Callahan, William Shell, Quantitative method and apparatus for measuring QT intervals from ambulatory electrocardiographic recordings.

In the above sources, a general and typical peculiarity of the described methods and devices is analysis of duration of selected time intervals of the cardiac cycle based on recording of electric components of cardiac electromagnetic signals by establishing a difference of potentials on human body surface (electrocardiogram - ECG), further processing and extracting moments of beginning and end of QRS, QT and ST-T intervals selected for analysis. Complex laws of changing parameters of R and T wave forms depending on mechanisms and pathology of myocardium lesion restrict the opportunities of these methods and devices in many ways.

The QT-interval measurement method in electrocardiogram and suitable device [US Patent 7,627,369, A61B5/0452, 2009, Hunt, Anthony Charles (Devon, GB), Qt-Interval Measurement in the Electrocardiogram, Int.] are the most proximate to the claimed invention.

The device contains the following modules: signal recording module, whose output is connected to the signals preliminary processing module, containing analogous and digital transformers, filters and processed signals accumulating unit and connected to the signals transformation and analysis module, joined to the QT, QRS and ST-T-intervals calculation module.

Using the signal recording module, contacting with human body surface through electrodes in specified areas, change of potential component of cardiac electromagnetic signal is recorded. Signals from the signal recording module output enter the signal preliminary processing module, where data analysis and recognition is performed using a set of special algorithms and programs of beginning and end time points of cardiac cycle intervals, selected for analysis. Final stage of signals processing is implemented using the QT, QRS and ST-T-intervals calculation module.

However, an essential restriction for precision and possibility of clinical use of such method and device for measuring cardiac cycle QT, QRS and ST-T intervals is recording of cardiac electromagnetic signals electric component and further analysis of a curve of this component's values change within the cardiac cycle itself. Measuring potential signals, doctors deal with combined action potential instead of indicators of electrophysiological state of myocardium's separate areas, in case of discordant leads - with a projection of heart's electric axis global vector, which almost excludes topographical selectivity in reflecting heart's electric activity. All this materially restricts possibilities of diagnosis using electropotential measurement.

The invention is based on a task of increasing precision and reliability of cardiac cycle QT, QRS and ST-T-intervals measurement not depending on the law of their changes and pathological lesions.

The second task put in the basis of the invention is development of a device to measure cardiac cycle QT, QRS and ST-T-intervals.

The set task is implemented as follows: in the measurement method of QT, QRS, ST-T-of cardiac cycle intervals, under which cardiac electromagnetic signals are recorded using the cardiac electromagnetic signals recording module, signals preliminary processing is then performed by averaging and preparing for analysis using the signals preliminary processing module; then data are analyzed and recognized using the signals transformation and analysis module, and final processing is performed using the QT, QRS, ST-T-of cardiac cycle intervals' duration calculation module, which serve as markers of myocardium's electric instability, *in accordance with the invention,* during cardiac electromagnetic signals recording magnetic sensors of the cardiac electromagnetic signals recording module are used, isolating the magnetic component of cardiac electromagnetic signals, and magnetic field changes are recorded in one plane above the patient's chest, during data analysis and recognition using the signals transformation and analysis module single-step magnetic field maps are plotted using recorded and pre-processed heart's magnetic signals, and a" inverse problem solutions" is used, plotting single-step maps of current density vectors distribution of sources emitting the recorded magnetic signals, while every map is represented with a time interval not exceeding 4 milliseconds, and prior to final processing, using the global current density calculation module, single-step global current density values during the time interval of the whole cardiac cycle or its separate areas are calculated for each current density vectors distribution map, at final processing, calculating the duration of QT, QRS, ST-T-of cardiac cycle intervals; the time interval beginning and end are considered the point of time when the curve of global current density value change reaches or crosses the zero values line.

The second set task is solved as follows: the QT, QRS and ST-T-of cardiac cycle intervals measurement device, containing the successively connected cardiac electromagnetic signals recording module, signals preliminary processing module, artifacts removal, averaging, computer memory accumulation, including low and high frequency program filters, signals transformation and analysis module and QT, QRS and ST-T-intervals duration calculation module, *in accordance with the invention,* additionally contains the global current density calculation module, whose input is connected to the output of the signals transformation and analysis module, and output - to the input of the QT, QRS i ST-T-intervals calculation module, the cardiac electromagnetic signals recording module additionally contains magnetic sensors for recording the magnetic component of cardiac electromagnetic signals, and the signals transformation and analysis module is developed with a possibility to plot magnetic field maps and current density vectors distribution maps for each moment of the cardiac cycle.

Using the method and device for magnetic field measurement in one plane above the patient's chest instead of potential electric signals measurement allow expanding the opportunities for more precise recognition of time changes in the cardiac cycle areas terms, and enhancing reliability of determining time parameters of QT, QRS i ST-T-of cardiac cycle intervals independent on the law of their changes and pathological lesions, which, in turn, contributes to more efficient diagnosing of human cardiovascular diseases, enabling for adequate therapeutic actions in patients with discovered increased myocardium's electric instability.

The invention is explained by a scheme depicting the device for measuring QT, QRS ST-T-of cardiac cycle intervals, including successively connected modules 1, 2, 3, 4, 5.
Module 1 is cardiac electromagnetic signal recording module, containing low and high frequency program filters, narrowband filters and adaptive noise compensation filters, magnetic sensors for recording the magnetic component of cardiac electromagnetic signals.
Module 2 is signal preliminary processing module, artifacts removal, averaging, computer memory accumulation, including low and high frequency program filters.
Module 3 is signals transformation and analysis module.
Module 4 is global current density calculation module.
Module 5 is QT, QRS and ST-T-intervals calculation module.

The method is implemented as follows.

Magnetic field above the patient's chest is measured instead of using magnetic sensors sensitive to the magnetic component of cardiac electromagnetic signals (module 1). Signals are subsequently entered, measurement results in the database are saved, and signal digital transformation is performed. Data processing consists of noise removal with subsequent digital filtration using low and high frequency program filters, narrowband filters and adaptive noise compensation filters. During data processing, synchronization with electrocardiogram is performed with breaking down the data into cardiac cycles, unnecessary cardiac cycles are removed, data averaging is performed (module 2). Equi-induction magnetocardiogram (MCG) maps are plotted and the "inverse problem solution" is used in module 3. Final results of MCG mapping are represented as current density vectors (CDV) distribution maps in a plane parallel to the frontal one. Each single CDV obtained due to solving of the "inverse problem solution" has its direction, size and position on the frontal plane. Further signal transformation is performed using module 4. In addition to analysis of single CDV maps, also global current density changes within cardiac cycle intervals, selected for analysis, in module 4 are analysed. Final stage of QT, QRS and ST-T-intervals calculation using module 5 is performed.

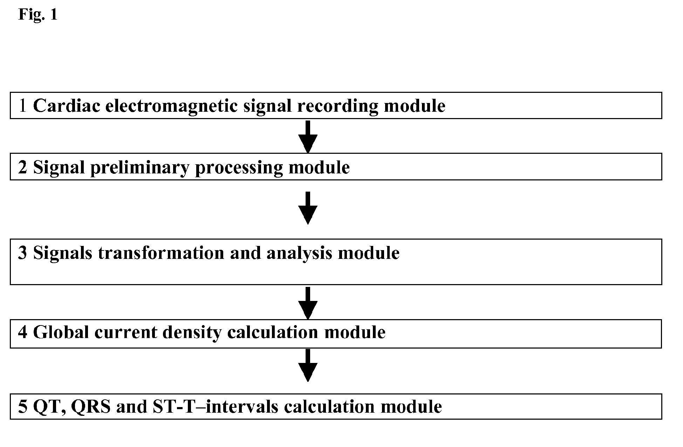

## Claims

1. Measurement method of QT, QRS, ST-T-of cardiac cycle intervals, under which cardiac electromagnetic signals are recorded using the cardiac electromagnetic signals recording module, signals preliminary processing is then performed by filtration, averaging and preparing for analysis using the signals preliminary processing module; then data are analyzed and recognized using the signals transformation and analysis module, and final processing is performed using the QT, QRS, ST-T-of cardiac cycle intervals' duration calculation module, which serve as markers of myocardium's electric instability, *distinguished in a way that* during cardiac electromagnetic signals recording magnetic sensors of the cardiac electromagnetic signals recording module are used, isolating the magnetic component of cardiac electromagnetic signals, and magnetic field changes are recorded in one plane above the patient's chest, during data analysis and recognition using the signals transformation and analysis module single-step magnetic field maps are plotted using recorded and pre-processed heart's magnetic signals, and a " inverse problem solution" is solved, plotting single-step maps of current density vectors distribution of sources emitting the recorded magnetic signals, while every map is represented with a time interval not exceeding 4 milliseconds, and prior to final processing, using the global current density calculation module, single-step global current density values during the time interval of the whole cardiac cycle or its separate areas are calculated for each current density vectors distribution map, at final processing, calculating the duration of QT, QRS, ST-T-of cardiac cycle intervals; the time interval beginning and end are considered the point of time when the curve of global current density value change reaches or crosses the zero values line.

2. Device for measuring QT, QRS and ST-T-of cardiac cycle intervals, containing the successively connected cardiac electromagnetic signals recording module, signals preliminary processing module, artifacts removal, averaging, computer memory accumulation, including low and high frequency program filters, signals transformation and analysis module and QT, QRS and ST-T-intervals duration calculation module, *distinguished in a way that* it additionally contains the global current density calculation module, whose input is connected to the output of the signals transformation and analysis module, and output - to the input of the QT, QRS i ST-T-intervals calculation module, the cardiac electromagnetic signals recording module additionally contains magnetic sensors for recording the magnetic component of cardiac electromagnetic signals, and the signals transformation and analysis module is developed with a possibility to plot magnetic field maps and current density vectors distribution maps for each moment of the cardiac cycle.
